# EUROPÄISCHE PATENTANMELDUNG

(11) **EP 2 792 387 A1**
(43) Veröffentlichungstag der Anmeldung: **22.10.2014**
(21) Anmeldenummer: 14001381.4
(22) Anmeldetag: 16.04.2014
(51) Int. Cl.: A61N 5/00, F21V 7/04

(54) **Ganzkörper-Belichtungseinheit**

(30) Priorität: 16.04.2013 DE 202013003573 U
(71) Anmelder: Saalmann, Gerhard, 32049 Herford (DE)
(72) Erfinder: Saalmann, Gerhard, 32049 Herford (DE)

(57) **Zusammenfassung**

Die vorliegende Erfindung betrifft eine Bestrahlungsvorrichtung (1) zum Bestrahlen eines Benutzers mit aktinischer Strahlung, umfassend ein quaderförmiges Gehäuse (11) mit fünf Wänden, wobei die fehlende sechste Wand des Quaders die Längswand ist, in deren Richtung - gesehen von der zentralen Längsachse (LA) des Quaders - aktinische Strahlung emittiert wird von wenigstens einer austauschbaren, aktinische Strahlung mindestens einer Wellenlänge emittierenden Lichtquelle (12a) und die von der wenigstens einen Lichtquelle (12a) emittierte aktinische Strahlung reflektiert wird von einem Reflektor (13) mit mehreren in einer Prisma-Anordnung zueinander angeordneten Reflektorflächen (13a, 13b, 13c, 13d, 13e), von denen mindestens zwei einen Durchlass oder mehrere Durchlässe (14) für Halterungen und Anschlüsse zur Steuerung der wenigstens einen Lichtquelle (12a) und deren Versorgung mit elektrischem Strom aufweisen; wobei in dem quaderförmigen Gehäuse (11) Vorschalteinrichtungen, Steuereinrichtungen und Anschlüsse für die Stromversorgung der wenigstens einen Lichtquelle (12a) der Bestrahlungsvorrichtung (1) untergebracht sind; und Einrichtungen (15) zum Positionieren der Bestrahlungsvorrichtung (1) relativ zu einem Benutzer.

## Beschreibung

Die vorliegende Erfindung betrifft eine Ganzkörper-Belichtungseinheit für die Phototherapie. Insbesondere betrifft die vorliegende Erfindung eine Belichtungseinheit für die Phototherapie, mit der der Körper eines Patienten, also eines Menschen, der einer Phototherapie bedarf, als Ganzes bestrahlt werden kann oder wahlweise auch teilweise, also nur hinsichtlich einzelner Körperpartien, bestrahlt werden kann. Für eine solche Bestrahlung kann das Bestrahlungspektrum oder die Bestrahlungs-Wellenlänge bzw. ein enger Bestrahlungs-Wellenlängenbereich durch Einsetzen von für eine bestimmte Phototherapie passenden Bestrahlungslampen in die Ganzkörper-Belichtungseinheit gemäß der Erfindung vorgegeben werden.

Die Bestrahlung des menschlichen Körpers mit aktinischer Strahlung eines mehr oder weniger großen Wellenlängenbereichs zur Erzielung einer therapeutischen Wirkung hat eine bereits auf die Mitte des vorigen Jahrhunderts zurückgehende Geschichte. In Kliniken und Sanatorien, jedoch auch im häuslichen Bereich, wurden Menschen und insbesondere Kinder und Kranke sogenannten "Höhensonnen" ausgesetzt, die Ultraviolettstrahlung und Infrarotstrahlung emittierten und damit bakterizide (beispielsweise Akne-Symptome bekämpfende) oder vitaminogene (beispielsweise Vitamin D₂ (Cholecalciferol) und Vitamin D₃ (Ergocalciferol) aus ihren Vorstufen 7-Dehydrocholesterol bzw. Ergosterol synthetisierende und eine die Knochencalcifizierung fördernde) Wirkung ausübten.

Der vorstehend beschriebenen direkten Wirkung von Strahlung bestimmter Wellenlänge auf biochemische und physiologische Prozesse wurde später die sogenannte "photodynamische Therapie" zur Seite gestellt: Bei der "photodynamischen Therapie" wird Licht bestimmter Wellenlängen (-bereiche) oder sogar eines schmalbandigen Spektrums oder einer einzigen Wellenlänge auf den Körper eingestrahlt und trifft auf photosensibilisierbare Wirkstoffe, die - in Einzelfällen sogar erst als Vorstufe/Prodrug - in einem erkrankten Gewebe angereichert sind. Vorstufen und Prodrugs werden in den jeweiligen Photosensibilisator umgewandelt, der von der Strahlung in einen angeregten Zustand überführt wird und nach einer - stoffspezifischen - Lebensdauer des angeregten Zustandes unter Abgabe therapeutisch nutzbarer Energie einer konkreten Wellenlänge/Energie in den Grundzustand zurückkehrt. Mittels aktinischer Strahlung beispielsweise im sichtbaren grünen Bereich (Wellenlängen von 540 bis 550 nm) können so mit Protoporphyrin IX als Photosensibilisator Psoriasis arthropatica und andere arthritische Beschwerden wirksam behandelt werden, siehe EP-B 1 583 525.

Problematisch war im Zusammenhang mit derartigen Behandlungen die Bereitstellung einer Vorrichtung, die sowohl im klinischen als auch im häuslichen Bereich einsetzbar ist, leicht aufzustellen und zu bedienen ist und eine für die Phototherapie geeignete Strahlung der gewünschten Wellenlänge zu liefern in der Lage ist. Bisher bekannte Vorrichtungen waren nicht nur sehr groß (und daher selbst für eine Verwendung in Kliniken und Sanatorien oft nicht ideal geeignet), sondern auch schwierig zu bedienen, nicht ausreichend sicher für eine Verwendung mit Strahlung bzw. gegen eine Überdosierung von Strahlung und nicht variabel hinsichtlich gewünschter Wellenlängen, insbesondere bei einer photodynamischen Therapie, bei der eine genaue Abstimmung der Wellenlänge des eingestrahlten Lichts mit dem jeweiligen zu verwendenden Photosensibilisator erforderlich ist.

Aufgabe der vorliegenden Erfindung war es, eine sowohl für die Ganzkörperbestrahlung als auch für die Teilkörperbestrahlung geeignete Belichtungseinheit bereitzustellen.

Eine weitere Aufgabe der Erfindung war, eine Belichtungseinheit bereitzustellen, in der für die jeweilige Bestrahlungs-"Aufgabe" eine passende Bestrahlungslampe eingesetzt werden kann, also eine Bestrahlungslampe, die in der Lage ist, aktinische Strahlung der gewünschten Wellenlänge oder des gewünschten Wellenlängen-Bandes zu emittieren, auch (aber nicht allein) als Ergebnis eines schnellen Austauschs der verwendeten Bestrahlungslampen.

Eine weitere Aufgabe der Erfindung war, eine Bestrahlungseinheit für die Ganzkörperbestrahlung oder Teilkörperbestrahlung bereitzustellen, die leicht und sicher bedient, d. h. eingeschaltet, hinsichtlich der Bestrahlungszeit programmiert und bei Bedarf auch von Hand ausgeschaltet werden kann.

Eine weitere Aufgabe der Erfindung war, eine Bestrahlungseinheit bereitzustellen, die auch im häuslichen Bereich Verwendung finden kann, also sowohl hinsichtlich Sicherheit als auch hinsichtlich Aufbau, Abbau, Transport und zwischenzeitlicher Lagerung an eine Verwendung im privaten Umfeld angepaßt ist.

Überraschenderweise wurden die vorstehenden und weitere dem Fachmann ohne Weiteres ersichtliche Aufgaben gelöst durch die vornehmlich (aber nicht beschränkend) für eine Ganzkörper-Bestrahlung geeignete Bestrahlungsvorrichtung gemäß der Erfindung mit mindestens einem wenigstens eine Lichtquelle oder Bestrahlungslampe in einem quaderförmigen Gehäuse mit einem Reflektor sowie Vorschalteinrichtungen und Steuerungseinrichtungen umfassenden Bestrahlungsmodul.

Die Erfindung betrifft daher eine Bestrahlungsvorrichtung zum Bestrahlen eines Benutzers mit aktinischer Strahlung, umfassend
- ein quaderförmiges Gehäuse mit fünf Wänden, wobei die fehlende sechste Wand des Quaders die Längswand ist, in deren Richtung - gesehen von der zentralen Längsachse des Quaders -
- aktinische Strahlung emittiert wird von wenigstens einer austauschbaren, aktinische Strahlung mindestens einer Wellenlänge emittierenden Lichtquelle und
- die von der wenigstens einen Lichtquelle emittierte aktinische Strahlung reflektiert wird von einem Reflektor mit mehreren in einer Prisma-Anordnung zueinander angeordneten Reflektorflächen, von denen mindestens eine einen Durchlass oder mehrere Durchlässe für Halterungen und Anschlüsse zur Steuerung der wenigstens einen Lichtquelle (und deren Versorgung mit elektrischem Strom aufweisen;
- wobei in dem quaderförmigen Gehäuse Vorschalteinrichtungen, Steuereinrichtungen und Anschlüsse für die Stromversorgung der wenigstens einen Lichtquelle der Bestrahlungseinrichtung untergebracht sind; und
- Einrichtungen zum Positionieren der Bestrahlungsvorrichtung relativ zu einem Benutzer.

Bevorzugte Ausführungsformen der Bestrahlungsvorrichtung gemäß der Erfindung sind in den abhängigen Ansprüchen 2 bis 11 beansprucht.

Die Erfindung betrifft weiter Ganzkörper-Bestrahlungseinheiten und Teilkörper-Bestrahlungseinheiten, die wenigstens eine der Bestrahlungsvorrichtungen zum Bestrahlen eines Benutzers mit aktinischer Strahlung wenigstens einer Wellenlänge sowie eine oder mehrere Einrichtung(en) zum Transportieren, Sichern, Aufstellen, Betreiben und/oder Abschirmen der mindestens einen Bestrahlungsvorrichtung gemäß Anspruch 12 oder 17 umfassen.

Bevorzugte Ausführungsformen der Ganzkörper-Bestrahlungseinheiten sind in den Ansprüchen 13 bis 16 beansprucht.

Die Erfindung ist hinsichtlich bevorzugter Ausführungsformen in den nachfolgenden, die Erfindung nicht beschränkenden Figuren gezeigt, die dem besseren Verständnis der Erfindung dienen sollen. Es zeigen:
Figuren 1 A, 1B und 1C drei verschiedene Ausführungsformen einer Bestrahlungsvorrichtung gemäß der Erfindung;
Figuren 2A, 2B und 2C drei Ansichten einer Bestrahlungsvorrichtung gemäß der Erfindung in der in den Figuren 1A und 1 B gezeigten Ausführungsform; Figur 2A zeigt eine rückwärtige, teilweise geschnittene Ansicht; Figur 2B zeigt eine Frontansicht, und Figur 2C zeigt eine seitliche, teilweise geschnittene Ansicht;
Figuren 3A, 3B und 3C drei Ansichten einer Bestrahlungsvorrichtung gemäß der Erfindung in der in Figur 1C gezeigten Ausführungsform; Figur 3A zeigt eine rückwärtige, teilweise geschnittene Ansicht; Figur 3B zeigt eine Frontansicht, ebenfalls teilweise geschnitten, und Figur 3C zeigt eine seitliche, teilweise geschnittene Ansicht;
Figur 4 eine aus zwei an den Endflächen aufeinandergesetzten Bestrahlungsvorrichtungen gemäß der Erfindung aufgebaute Ganzkörper-Bestrahlungseinheit gemäß der Erfindung in perspektivischer Ansicht;
Figur 5 eine zum gemeinsamen Betreiben mehrerer Bestrahlungsvorrichtungen gemäß der Erfindung in Ganzkörper-Bestrahlungseinheiten gemäß der Erfindung verwendbare Kabelverbindung;
Figur 6 eine aus zwei Bestrahlungsvorrichtungen aufgebaute Ganzkörper-Bestrahlungseinheit gemäß der Erfindung im Transportzustand in perspektivischer Ansicht, in der die beiden Bestrahlungsvorrichtungen gegeneinander geklappt und für einen Transport gesichert und mit einer Trageeinrichtung wie beispielsweise einem Tragegriff versehen gezeigt sind;

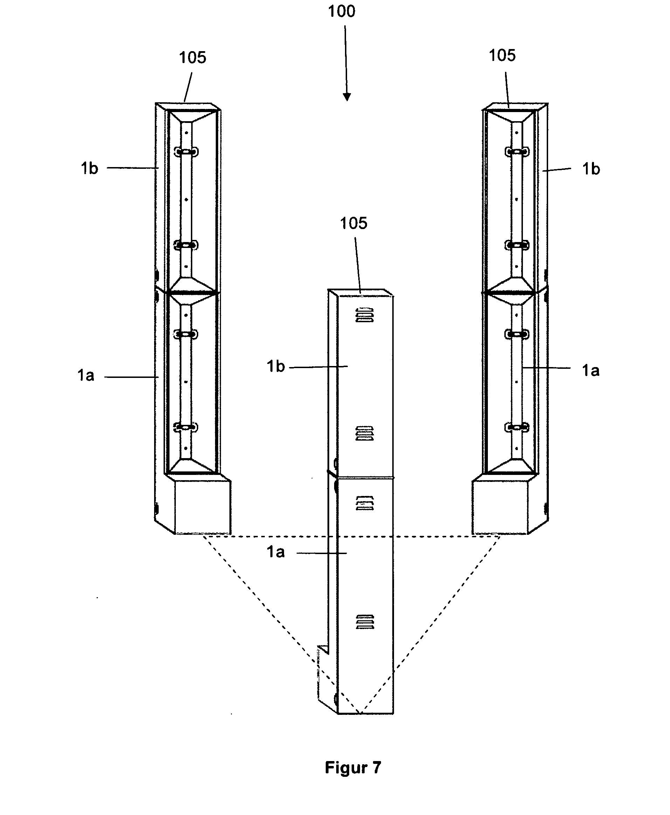

Figur 7 eine perspektivische Ansicht einer aus drei Paaren von Bestrahlungsvorrichtungen aufgebaute Ganzkörper-Bestrahlungseinheit gemäß der Erfindung, in der die drei Paare von Bestrahlungsvorrichtungen gemäß der Erfindung in einem festen Abstand zueinander in Positionen aufgestellt sind, die den Eckpunkten eines Dreiecks entsprechen, und hinsichtlich der Strahlungsrichtung so angeordnet sind, daß die Bestralungsvorrichtungen der jeweils drei Paare von Bestrahlungsvorrichtungen so angeordnet sind, daß sie auf ein imaginäres Zentrum des Dreiecks hin strahlen, in dem im Betrieb ein Benutzer positioniert sein kann, der bestrahlt werden soll;

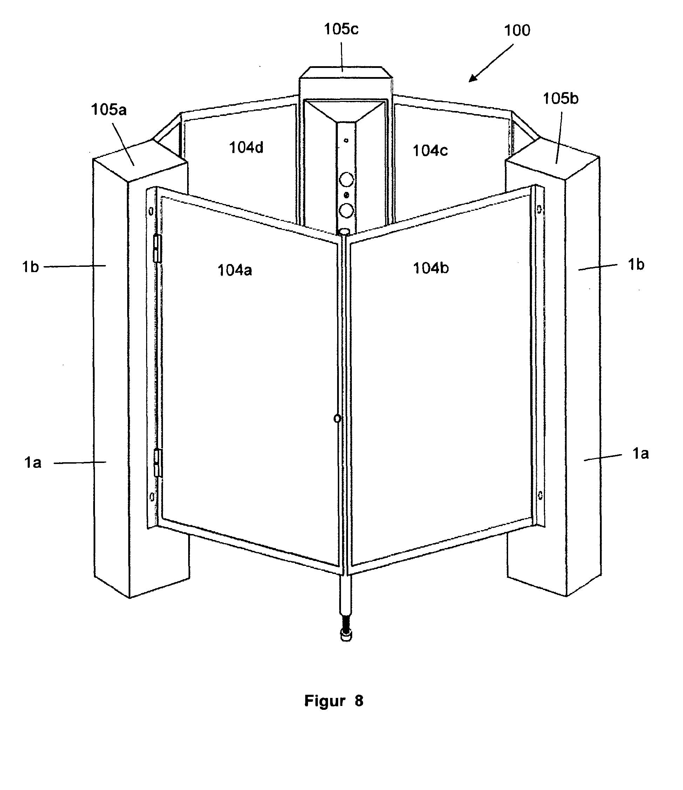

Figur 8 eine Figur 7 entsprechende Ansicht einer aus drei Paaren von Bestrahlungsvorrichtungen aufgebaute Ganzkörper-Bestrahlungseinheit gemäß der Erfindung, in der die drei Paare von Bestrahlungsvorrichtungen gemäß der Erfindung in einem festen Abstand zueinander in Positionen aufgestellt sind, die den Eckpunkten eines Dreiecks entsprechen, und hinsichtlich der Strahlungsrichtung so angeordnet sind, daß die Bestralungsvorrichtungen der jeweils drei Paare von Bestrahlungsvorrichtungen so angeordnet sind, daß sie auf ein imaginäres Zentrum des Dreiecks hinstrahlen, in dem im Betrieb ein Benutzer positioniert sein kann, der bestrahlt werden soll, und in der die drei Paare von erfindungsgemäßen Bestrahlungsvorrichtungen Stellwänden als Einrichtungen zum Abschirmen der aktinischen Strahlung und Behandlungsraum zur Wahrung der Intimität des Patienten verbunden sind, wodurch ein mobiler Behandlungsraum zur Bestrahlungsbehandlung geschaffen wird;
Figur 9 eine eine Bestrahlungsvorrichtung gemäß der Erfindung umfassende Teilkörper-Bestrahlungseinheit mit einem Pendelhalter zum sicheren Aufstellen; und
Figur 10 ein beispielhaftes Handbedienungsgerät für eine Bestrahlungsvorrichtung oder eine Ganz- oder Teilkörper-Bestrahlungseinheit gemäß der Erfindung.

Die Erfindung wird nun nachfolgend unter Bezugnahme auf die beigefügten Figuren näher beschrieben. Die beigefügten Figuren sowie die detaillierte Beschreibung dienen jedoch lediglich der beispielhaften Beschreibung der Erfindung und sollen deren besseres Verständnis ermöglichen. Keinesfalls dienen die Figuren und/oder die nachfolgende Beschreibung der bevorzugten Ausführungsformen der Erfindung einer Beschränkung der Erfindung auf die beschriebenen, möglicherweise speziellen Ausführungsformen.

Des Weiteren können besondere Ausführungsformen der Erfindung Kombinationen einzelner Merkmale der Erfindung, Kombinationen mehrerer Merkmale der Erfindung oder eine Kombination aller Merkmale der Erfindung betreffen; die im Detail beschriebenen Merkmale sind einzeln kombinierbar oder zu mehreren kombinierbar oder alle gemeinsam kombinierbar zur Erzielung besonderer Vorteile der Erfindung.

Die Erfindung betrifft eine Bestrahlungsvorrichtung 1 gemäß der Erfindung, deren Zweck es ist, einen Benutzer gezielt mit aktinischer Strahlung zu bestrahlen, der eine Bestrahlung mit aktinischer Strahlung wünscht oder einer Bestrahlung mit aktinischer Bestrahlung bedarf, beispielsweise (aber nicht beschränkend) aus medizinischen Gründen.

Unter dem Begriff "aktinische Strahlung", wie er in der vorliegenden Beschreibung und in den Patentansprüchen verwendet wird und gegebenenfalls auch einfach und kurz mit "Strahlung" angegeben wird, wird für die Zwecke der vorliegenden Erfindung jegliche Lichtstrahlung natürlichen oder künstlichen Ursprungs verstanden, mit der photochemische oder speziell photobiologische oder photomedizinische Wirkungen erzielt werden können. In spezielleren Ausführungsformen, die die Erfindung jedoch nicht beschränken, wird unter "aktinischer Strahlung" Lichtstrahlung verstanden, die photochemische und/oder photobiologische und/oder photomedizinische Wirkungen erzielen kann. Solche aktinische Strahlung kann stammen aus dem Bereich UV-Lichtstrahlung und/oder sichtbare Lichtstrahlung und/oder IR-Lichtstrahlung, die für den Säugerkörper, speziell den Körper eines Menschen, zu vorteilhaften photochemischen und/oder photobiologischen und/oder photomedizinischen Wirkungen führt. Die von der erfindungsgemäßen Bestrahlungsvorrichtung emittierte aktinische Strahlung ist dabei nicht auf irgendwelchen speziellen Wellenlängen oder Bereiche ("Bänder") von Wellenlängen beschränkt.

Die aktinische Strahlung kann Strahlung einer einzigen Wellenlänge sein oder kann Strahlung eines mehr oder weniger breiten Bandes von Wellenlängen sein oder kann auch Bänder von Wellenlängen umfassen, die sich über einen mehr oder weniger großen Bereich von Wellenlängen erstrecken, die aus einem der vorgenannten Bereiche oder auch aus mehreren der vorgenannten Bereiche UV-Lichtstrahlung, sichtbare Lichtstrahlung und IR-Lichtstrahlung stammen kann.

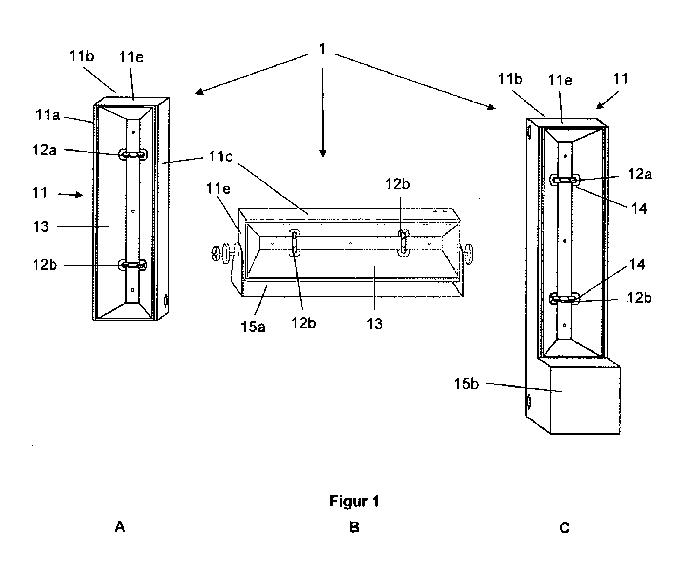

Erfindungsgemäß kann eine einzelne Wellenlänge oder ein mehr oder weniger breites Bereich (Band) von Wellenlängen entsprechend den Erfordernissen der Bestrahlung aus allen denkbaren Bereichen von Wellenlängen gewählt werden, die der Fachmann für die Bestrahlung von Säugern und speziell von Menschen kennt. In bevorzugten Ausführungsformen der Erfindung ist/sind der Wellenlängenbereich oder die Wellenlängenbereiche gewählt aus der Gruppe Wellenlängenbereich UV-Lichtstrahlung und/oder Wellenlängenbereich sichtbare Lichtstrahlung und/oder Wellenlängenbereich IR-Lichtstrahlung, vorzugsweise aus der Gruppe Wellenlängenbereich von 285 nm bis 400 nm, Wellenlängenbereich von 400 bis 800 nm und/oder Wellenlängenbereich von 800 nm bis 1200 nm; weiter bevorzugt gewählt ist/sind aus der Gruppe des UV-Wellenlängenbereiches von 285 nm bis 350 nm und/oder des UV-Wellenlängenbereiches von 350 nm bis 400 nm und/oder des sichtbaren Wellenlängenbereiches von 400 nm bis 460 nm (violett über blau bis grün), 460 nm bis 550 nm (grün bis gelb), 550 nm bis 680 nm (gelb bis rot) und 680 nm bis 800 nm (rot bis dunkelrot) und/oder der IR-Wellenlängenbereiches von 800 nm bis 1200 nm. Erfindungsgemäß umfaßt die Bestrahlungsvorrichtung 1 gemäß der Erfindung, wie sie beispielhaft in den Figuren 1A, 1B und 1C gezeigt ist, mehrere Komponenten.

Die Bestrahlungsvorrichtung 1 gemäß der Erfindung umfaßt gemäß dem Patentanspruch 1 als eine der Komponenten ein quaderförmiges Gehäuse 11. In besonderen Ausführungsformen der Erfindung kann die Form des Gehäuses 11 der Bestrahlungsvorrichtung 1 auch von der Quader-Form abweichen. Das quaderförmige Gehäuse 11 besteht aus drei Wänden 11 a, 11 b, 11 c in Richtung der Längsachse LA des Quaders und zwei Endwänden 11 e und 11 f. Von diesen haben die Seitenwände 11a und 11 c die gleiche Dimensionierung und haben die Endwände 11e und 11f die gleiche Dimensionierung. Die Rückwand 11 b des Quaders liegt keine Vorderwand gegenüber; an Stelle der Vorderwand weist das quaderförmige Gehäuse einen (nachfolgend im Einzelnen beschriebenen) Reflektor 13 mit wenigstens einer aktinisches Licht emittierenden Lichtquelle (12a), vorzugsweise mit zwei aktinisches Licht emittierenden Lichtquellen (12a, 12b) auf.

Die vorgenannten Wände 11 a, 11 b, 11 c, 11 e und 11f des quaderförmigen Gehäuses 11 der Bestrahlungsvorrichtung 1 gemäß der Erfindung können aus beliebigen, dem Fachmann für Zwecke der Bildung eines derartigen Strahler-Gehäuses bekannten und als geeignet erscheinenden Material sein. In bevorzugten Ausführungsformen der Erfindung umfaßt das quaderförmige Gehäuse 11 Wände aus einem Material, das gewählt ist aus leichten und Stabilität und Standfestigkeit erbringenden Materialien und Kombinationen von Materialien aus der Gruppe Holz, Kunststoff, Leichtmetalle, weiter bevorzugt Kunststoff und Leichtmetalle. Grundsätzlich ist es möglich, verschiedene Wände aus verschiedenen Materialien herzustellen. Aus Gründen einer einfachen und zweckmäßigen Herstellung des Gehäuses 11 der erfindungsgemäßen Bestrahlungsvorrichtung 1 ist es jedoch bevorzugt, die Wände des Gehäuses 11 aus einem Material oder einer Kombination von Materialien herzustellen. Besonders bevorzugt sind als Material(ien) für die Gehäusewände 11a, 11b, 11c, 11 e und 11 f des quaderförmigen Gehäuses 11 der Bestrahlungsvorrichtung 1 Kunststoffe und leichte Metalle und Verbünde bzw. Kombinationen hieraus.

Erfindungsgemäß ist eine weitere Komponente der erfindungsgemäßen Bestrahlungsvorrichtung wenigstens eine Lichtquelle 12a, die in der Lage ist, aktinische Strahlung zu emittieren. Es können in besonderen Ausführungsformen der Erfindung auch pro Bestrahlungsvorrichtung 1 mehr als eine Lichtquelle, beispielsweise zwei, drei oder vier Lichtquellen, verwendet werden; erfindungsgemäß bevorzugt ist jedoch die Verwendung von genau einer Lichtquelle 12a oder genau zweier Lichtquellen 12a, 12b (siehe Figuren 1A, 1B, 1C usw.), die in der Lage ist/sind, aktinische Strahlung zu emittieren. Die emittierte Strahlung kann dabei die Strahlung einer konkreten Wellenlänge, die Strahlung eines mehr oder weniger breiten Wellenlängen-Bandes oder auch über ein breites Wellenlängenband sich erstreckende Strahlung sein.

Erfindungsgemäß bevorzugt läßt sich die Lichtquelle 12a oder lassen sich die Lichtquellen 12a, 12b leicht austauschen. Dies kommt nicht nur in einem Fall zum Tragen, in denen eine Lichtquelle (oder auch beide Lichtquellen) defekt ist/sind und durch neue Lichtquellen des gleichen Typs wie vorher (also emittierend die gleiche Wellenlänge oder das gleiche Wellenlängen-Band) ersetzt werden muß/müssen. In einer bevorzugten Ausführungsform ist es auch durch die richtige Wahl der Fassungen und Anschlüsse möglich, Lichtquellen zum Emittieren von Licht einer Wellenlänge oder eines konkreten Bandes von Wellenlängen durch Lichtquellen einer anderen Wellenlänge oder eines anderen Bandes von Wellenlängen zu ersetzen. Dadurch kann die Verwendbarkeit der Bestrahlungsvorrichtungen 1 der Erfindung auf eine Vielzahl von Wellenlängen oder Wellenlängen-Bereichen oder -Bändern ausgedehnt werde und damit den jeweiligen Wünschen oder medizinischen oder kosmetischen Erfordernissen angepaßt werden.

In bevorzugten Ausführungsformen der Erfindung, die eine breite Verwendbarkeit der Bestrahlungsvorrichtungen 1 und der mit diesen bestückten Ganzkörper-oder Teilkörper-Belichtungseinheiten 100, 200 ermöglichen, umfassen die erfindungsgemäßen Bestrahlungsvorrichtungen 1 vorzugsweise eine aktinische Strahlung emittierende Lichtquelle 12a oder zwei, drei oder vier aktinische Strahlung emittierende Lichtquellen 12a, 12b, 12c, 12d. Weiter bevorzugt sind zwei, drei oder vier aktinische Strahlung des gleichen Wellenlängenbereichs emittierende Lichtquellen 12a, 12b, 12c, 12d. Wie oben bereits beschrieben, umfassen die Bestrahlungsvorrichtungen 1 gemäß der Erfindung noch weiter bevorzugt zwei Lichtquellen 12a, 12b, die aktinische Strahlung des gleichen Wellenlängenbereichs emittieren.

Alternativ ist es jedoch auch in weiteren, erfindungsgemäß bevorzugten Ausführungsformen der Bestrahlungsvorrichtungen 1 möglich, wenigstens zwei Lichtquellen 12a, 12b in einer Bestrahlungsvorrichtung 1 zu verwenden, die aktinische Strahlung in wenigstens zwei Wellenlängenbereichen emittieren. Damit kann vorteilhafterweise ein größeres Spektrum der Bestrahlungsbehandlung abgedeckt werden.

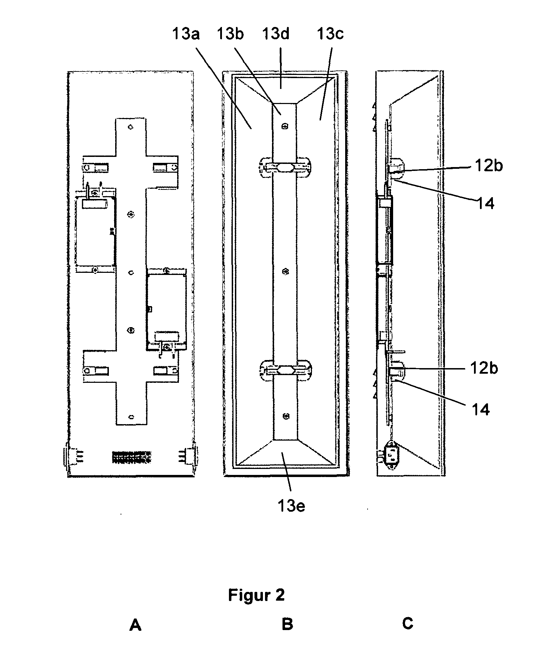

Erfindungsgemäß ist eine weitere, von der erfindungsgemäßen Bestrahlungsvorrichtung 1 umfaßte Komponente ein Reflektor 13, wie dies beispielsweise aus den Figuren 1A, 1B und 1C bzw. 2A, 2B und 2C bzw. 3A, 3B und 3C ersichtlich ist. Dieser Reflektor 13 weist erfindungsgemäß mehrere in einer Prisma-Anordnung zueinander angeordneten Reflektorflächen 13a, 13b, 13c, 13d, 13e auf.

Das Material des Reflektors kann von einem Fachmann in diesem Bereich nach an sich bekannten Kriterien einer optimalen Reflexion des von den Lichtquellen 12a, 12b emittierten Lichtes und möglicherweise auch nach Kriterien einer selektiven Reflexion von aktinischer Strahlung, die von den Lichtquellen emittiert wird, ausgewählt werden. In bevorzugten Ausführungsformen der Erfindung besteht der Reflektor 13 aus einem Material, das gewählt ist aus Metallen wie beispielsweise Aluminium oder Kunststoff(en) oder Gläsern, die mit einem oder mehreren Metall(en) wie beispielsweise Aluminium, Silber o. ä. beschichtet bzw. bedampft ist/sind. Besonders bevorzugt sind als Reflektormaterialien metallbeschichtete (beispielsweise Aluminium- oder Silber-beschichtete Kunststoffe oder Gläser, insbesondere hitzebeständige Gläser, auf die eines oder mehrere Metalle durch Bedampfung in dünnen Schichten aufgebracht ist/sind.

Zur Optimierung des Reflexions-Ergebnisses weist in bevorzugten Ausführungsformen der Erfindung der Reflektor 13 fünf Reflektorflächen 13a, 13b, 13c, 13d, 13e auf. Dies ist im Einzelnen auch aus den Figuren 1A, 1B und 1C bzw. 2B und 2C bzw. 3B und 3C ersichtlich. Von den fünf Reflektorflächen 13a, 13b, 13c, 13d und 13e erstrecken sich eine zentrale rechtwinklige Reflektorfläche 13b und zwei flankierende trapezförmige Reflektorflächen 13a, 13c in Richtung parallel zu der Quader-Längsachse LA , d.h. parallel zu den beiden Seitenwänden 11 a und 11 c und der Rückwand 11 b. Diese drei Reflektorflächen 13a, 13c und 13b sind in einem Winkel zueinander angeordnet, der einem gleichschenkligen Trapez entspricht. Dies ist beispielsweise aus den Figuren 1A, 1B, 1C, 2B, 2C, 3B und 3C leicht ersichtlich. Zwei weitere trapezförmige Reflektorflächen 13d, 13e erstrecken sich - wie ebenfalls aus den vorgenannten Figuren ersichtlich ist - von den oberen bzw. unteren Quader-Endflächen zu den Oberkanten bzw. Unterkanten der zentralen rechteckigen Reflektorfläche 13b und der beiden trapezförmigen Reflektorflächen 13a, 13c. So wird ein das Licht der Lichtquellen 12a, 12b zentral fokussierendes Reflexionsmuster gebildet, das einer gleichmäßigen Bestrahlung des Benutzers mit aktinischer Strahlung der gewünschten Wellenlängen des aktinischen Lichts dient. Bei der Bestrahlung bestimmter Krankheitsbilder können die Refexions- und Abstrahlungswinkel entsprechend dem Krankheitsbild angepaßt werden.

In der erfindungsgemäßem Bestrahlungsvorrichtung 1 sind die Halterungen, Kontakte und Anschlüsse der Lichtquelle(n) 12 sowie die Vorschaltgeräte und Kabelverbindungen für die Versorgung der Lichtquelle(n) 12 mit elektrischer Energie aus ersichtlichen, dem Fachmann bekannten Gründen so angeordnet, daß sie weder der von der Lichtquelle 12 bzw. von den Lichtquellen 12 direkt emittierten aktinischen Strahlung noch der von dem Reflektor 13 reflektierten aktinischen Strahlung im Wege sind bzw. von dieser auf die Dauer beeinträchtigt werden. Idealerweise sind diese in dem quaderförmigen Gehäuse 11 in dem Raum "hinter" dem Reflektor 13 (also zwischen den Reflektor-Rückwänden und den fünf Wänden 11 a, 11 b, 11 c, 11e und 11f des Gehäuses 11) untergebracht.

Die Reflektorwände, besonders bevorzugt die "flankierenden" Reflektorwände 13a und 13 c, weisen dann lediglich einen Durchlass 14 oder mehrere Durchlässe 14, vorzugsweise mehrere Durchlässe 14, noch weiter bevorzugt zwei oder mehr Durchlässe 14, ganz besonders bevorzugt jeweils zwei Durchlässe 14a, 14b für Halterungen und Anschlüsse zur Steuerung der wenigstens einen Lichtquelle 12a oder der zwei oder mehreren Lichtquellen 12a, 12b und deren Versorgung mit elektrischem Strom auf. Diese sind als solche aus dem Stand der Technik dem Fachmann hinsichtlich Aufbau und Anordnung bekannt und bedürfen daher an dieser Stelle keiner weiteren detaillierten Beschreibung.

Erfindungsgemäß umfassen die Bestrahlungsvorrichtungen 1 als weitere Komponenten Einrichtungen 15 zum Positionieren der Bestrahlungsvorrichtung 1 relativ zu einem Benutzer, der die Bestrahlung empfangen soll. Solche Einrichtungen sind als solche dem Fachmann bekannt und können je nach den Erfordernissen vom Fachmann frei gewählt werden.

In besonders bevorzugten Ausführungsformen der Erfindung können solche Positionier-Einrichtungen 15 als getrennte (und damit auch entsprechend den Erfordernissen frei wählbare) Einrichtungen 15 entweder an der Bestrahlungsvorrichtung 1, beispielsweise an deren Gehäuse 11, befestigbar sein oder können integral einstückig mit dem Gehäuse 11 der Bestrahlungsvorrichtung 1 ausgeführt sein.

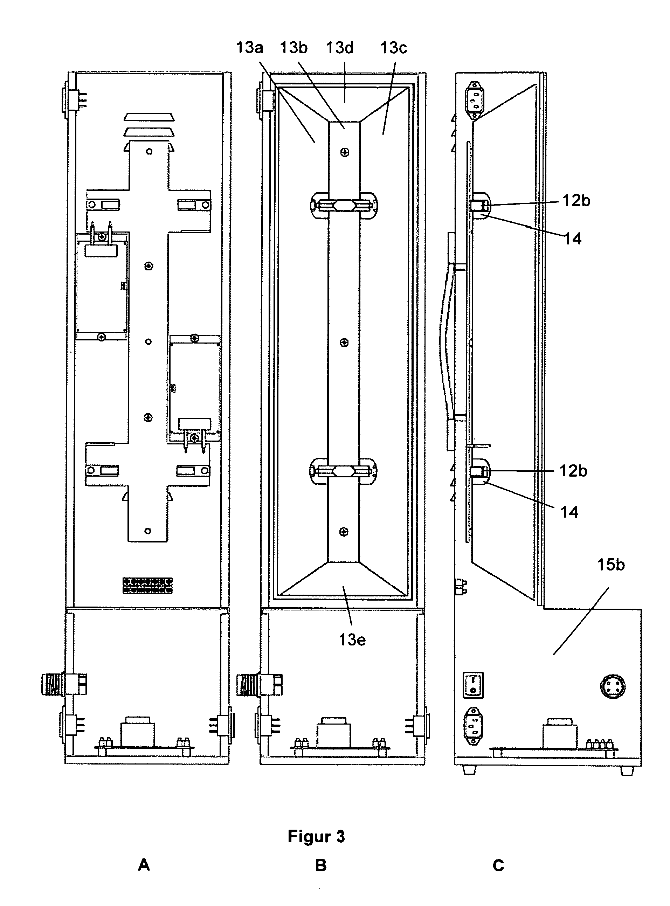

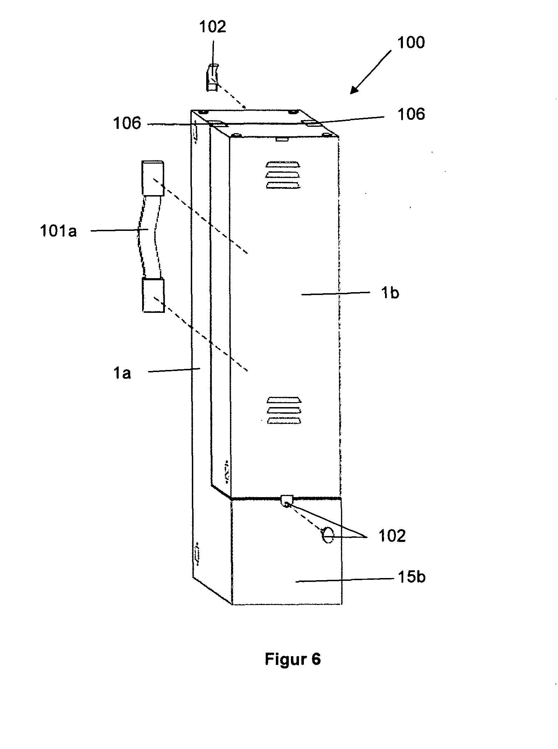

Positionier-Einrichtungen 15 der vorstehend zweitgenannten Art sind beispielsweise in den Figuren 1C, 3A, 3B, 3C, 4, 6 und 7 gezeigt und sind beispielsweise ein im Wesentlichen kubischer Standfuß 15b, ohne die Erfindung auf diesen zu beschränken. Ein solcher Standfuß 15b kann - wie beispielsweise in den Figuren 3A, 3B oder 3C gezeigt - neben der Positionierung der Bestrahlungsvorrichtung 1 auch dem Unterbringen eines oder mehrerer elektrischer Anschlüsse oder der Steuerung einer oder mehrerer Bestrahlungsvorrichtungen 1 gemäß der Erfindung dienen oder kann, wie in Figur 6 gezeigt, der Sicherung eines Paars gegeneinandergeklappter Bestrahlungsvorrichtungen 1, 1 beim Transport dienen. Weitere Funktionen des Standfußes 15b werden nachfolgend im Zusammenhang mit einer Ganzkörper-Bestrahlungseinheit 100 näher beschrieben.

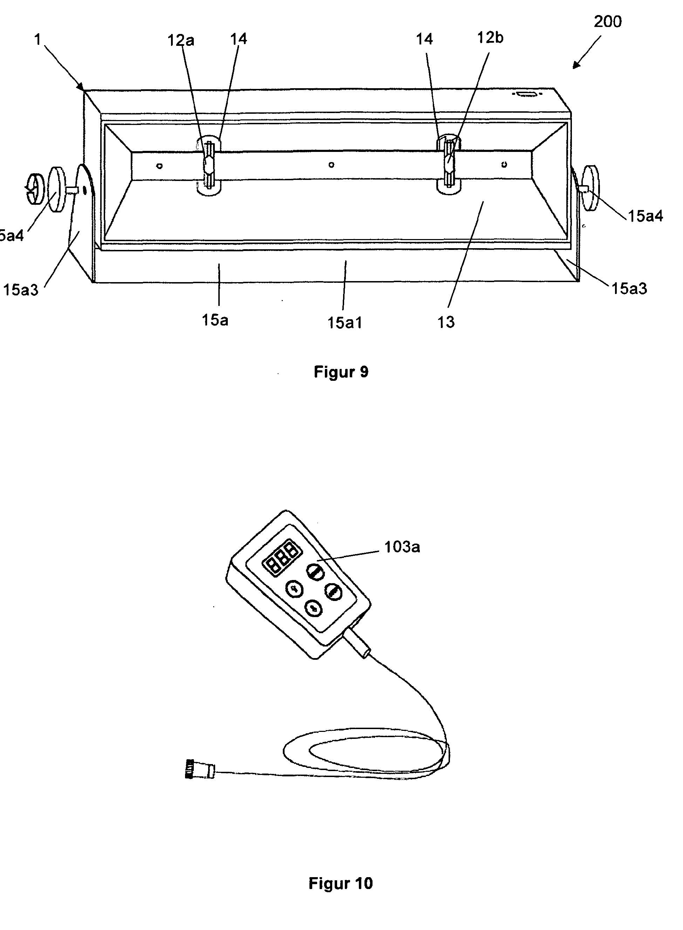

Positionier-Einrichtungen 15 der vorstehend erstgenannten Art sind Einrichtungen, mit denen eine Bestrahlungsvorrichtung 1 (oder auch mehrere Bestrahlungsvorrichtungen 1) gemäß der Erfindung relativ zu einem Benutzer positionert werden können, beispielsweise aufgestellt oder aufgehängt werden können. Ein bevorzugtes Beispiel einer derartigen Positionier-Vorrichtung 15 ist ein in Figur 9 beispielhaft gezeigter Pendelhalter 15a. Wie in Figur 9 gezeigt, kann eine Bestrahlungsvorrichtung 1 (oder können - nicht gezeigt - auch mehrere Bestrahlungsvorrichtungen 1) gemäß der Erfindung mittels einer solchen als Pendelhalter 15a ausgebildeten Positionier-Einrichtung aufgestellt werden, so daß die von der Lichtquelle 12a oder die von den Lichtquellen 12a, 12b emittierte aktinische Strahlung gezielt auf den Teil des Körpers eines Benutzers gerichtet werden kann, der bestrahlt werden soll. Alternativ kann eine Bestrahlungsvorrichtung 1 (oder können - nicht gezeigt - auch mehrere Bestrahlungsvorrichtungen 1) gemäß der Erfindung mittels einer solchen als Pendelhalter 15a ausgebildeten Positionier-Einrichtung aufgehängt werden, beispielsweise an einer vertikalen Fläche oder unter einer horizontalen Fläche, so daß die von der einen Lichtquelle 12a oder die von den mehreren (z. B. zwei) Lichtquellen 12a, 12b emittierte aktinische Strahlung gezielt auf den Teil des Körpers eines Benutzers gerichtet werden kann, der bestrahlt werden soll.

Dazu umfaßt vorzugsweise der Pendelhalter 15a eine rechtwinklige Halterfläche 15a1 mit einer Länge geringfügig über der Länge der Bestrahlungsvorrichtung. Diese dient als Standfläche bei Aufstellen des Pendelhalters 15a auf einer horizontalen Fläche mit der daran beweglich - beispielsweise über Rändelschrauben auf beiden Seiten drehbar - angebrachten Bestrahlungsvorrichtung 1 oder als Aufhängfläche bei Aufhängen des Pendelhalters 15a auf einer vertikalen Fläche oder unter einer horizontalen Fläche mit der daran beweglich - beispielsweise über Rändelschrauben auf beiden Seiten drehbar - angebrachten Bestrahlungsvorrichtung 1. Auf jeder der kurzen Seiten der Halterfläche 15a1 umfaßt der Pendelhalter 15a je eine im rechten Winkel zu der Halterfläche (15a1) angeordnete Halterlasche (15a2, 15a3) mit je einem Befestigungsmittel (15a4) zum reversiblen Befestigen der Bestrahlungsvorrichtung 1. Das Befestigungsmittel kann bevorzugt (nicht jedoch beschränkend) eine Rändelschraube (gegebenenfalls mit einer Sicherung) sein, die durch jeweils eine Öffnung in den Haltelaschen 15a2, 15a3 geführt wird und ein Schwenken der Bestrahlungsvorrichtung um die durch die beiden Schrauben gebildeten Drehachse und ein anschließendes reversibles Feststellen in Zusammenwirken mit herkömmlichen Feststellscheiben erlaubt. Dadurch läßt sich die optimale Position der Bestrahlungsvorrichtung relativ zu dem zu bestrahlenden Benutzer leicht und zuverlässig einstellen.

Bestrahlungsvorrichtungen 1 gemäß der Erfindung können gegebenenfalls weitere Komponenten enthalten, wie sie der Fachmann aufgrund seines Fachwissens kennt und erfahrungsgemäß vorsieht. Solche weiteren Komponenten können beispielsweise, jedoch nicht beschränkend, Lüftungsschlitze sein, die in einer der Wände oder in mehreren Wänden des quaderförmigen Gehäuses der Bestrahlungsvorrichtung angebracht sind. Beispiele solcher Lüftungsschlitze sind beispielsweise in den Figuren 6 und 7 zu erkennen, wo sie auf der rückwärtigen Wand des Gehäuses 11 angebracht sind.

Weiter können gegebenenfalls Ventilatoren für eine verbesserte Belüftung der Gehäuse und der in diesen untergebrachten Aggregate (Lichtquelle(n), Hilfsaggregate, Steuergeräte, Kabelverbindungen) sorgen.

Die Erfindung betrifft auch eine Ganzkörper-Belichtungseinheit 100 oder Teilkörper-Belichtungseinheit 200, umfassend mindestens eine Bestrahlungsvorrichtung 1 gemäß der vorstehenden detaillierten Beschreibung und eine oder mehrere Einrichtung(en) 101, 102, 103, 104 zum Transportieren, Sichern, Aufstellen, Betreiben und/oder Abschirmen der mindestens einen Bestrahlungsvorrichtung 1.

In erfindungsgemäß bevorzugten Ausführungsformen von Ganzkörper-Belichtungseinheiten 100 oder von der Teilkörper-Belichtungseinheiten 200 können die eine oder mehreren Einrichtung(en) 101, 102, 103, 104 zum Transportieren, Sichern, Aufstellen, Betreiben und/oder Abschirmen der mindestens einen Bestrahlungsvorrichtung 1 sein:
- als eine oder mehrere Einrichtung(en) 101 zum Transportieren eine oder mehrere Trage-Einrichtungen 101a, vorzugsweise ein oder mehrere Handgriff(e), besonders bevorzugt ein oder mehrere montierbare(n) und demontierbare(n) Handgriff(e) 101a; und/oder
- als eine oder mehrere Einrichtung(en) 102 zum Sichern eine oder mehrere Fixierlasche(n), Verschlußriegel, Magnetverschluß/-verschlüsse, Rändelmutter(n) 102; und/oder
- als eine oder mehrere Einrichtung(en) 106 zum Aufstellen ein oder mehrere Scharnier(e), Standfüße, Sicherungen 106a; und/oder
- als eine oder mehrere Einrichtung(en) 103 zum Betreiben ein oder mehrere Kabelverbindung(en), Handbedien-Gerät(e) 103a oder elektronische(s) Steuergerät(e); und/oder
- als eine oder mehrere Einrichtung(en) 104 zum Abschirmen eine oder mehrere für die emittierte Strahlung undurchlässige Abschirm-Einrichtung(en) 104a, vorzugsweise mobile Abschirmeinrichtung(en) 104a, 104b, 104c zur reversiblen Befestigung an der oder in der Nähe der einen oder mehreren Bestrahlungsvorrichtung(en) 1.

Was die Belichtungseinheit 1 als von der Ganzkörper-Belichtungseinheit 100 oder von der Teilkörper-Belichtungseinheit 200 umfaßte Komponente angeht, kann in vollem Umfang auf die vorstehende Beschreibung der bevorzugten Ausführungsformen der Bestrahlungsvorrichtung 1 und die Figuren, in denen einige der bevorzugten Ausführungsformen der Bestrahlungsvorrichtung 1 gezeigt werden, zurückgegriffen werden. Im Zusammenhang mit der erfindungsgemäßen Ganzkörper-Belichtungseinheit 100 oder der erfindungsgemäßen Teilkörper-Belichtungseinheit 200 wird also die Bestrahlungsvorrichtung nicht ein weiteres Mal beschrieben.

Die erfindungsgemäße Ganzkörper-Belichtungseinheit 100 oder Teilkörper-Belichtungseinheit 200 kann eine oder kann mehrere Bestrahlungsvorrichtungen 1 umfassen, wie sie oben im Einzelnen als erfindungsgemäß und als von bevorzugten Ausführungsformen der Erfindung umfaßt beschrieben wurden. In besonders bevorzugten Ausführungsformen der Erfindung umfassen Teilkörper-Bestrahlungseinheiten 200 gemäß der Erfindung vorteilhafterweise eine Bestrahlungsvorrichtung 1 gemäß der Erfindung und/oder umfassen Ganzkörper-Belichtungseinheiten 100 gemäß der Erfindung mindestens zwei Bestrahlungsvorrichtungen 1 gemäß der Erfindung, weiter bevorzugt Paare von (also Vielfache von zwei) Bestrahlungsvorrichtungen 1 gemäß der Erfindung, also zwei, vier, sechs usw. Bestrahlungsvorrichtungen 1.

Ein Beispiel einer Teilkörper-Bestrahlungseinheit 200 gemäß der Erfindung ist in Figur 9 gezeigt. Die in Figur 9 beispielhaft gezeigte Teilkörper-Bestrahlungseinheit 200 umfaßt mindestens eine, vorzugsweise genau eine, Bestrahlungsvorrichtung , wie sie vorstehend im Detail beschrieben wurde; und zum Positionieren der Bestrahlungsvorrichtung 1 relativ zu einem Benutzer einen Pendelhalter 15a zum Positionieren der Bestrahlungsvorrichtung 1, beispielsweise zum Aufstellen der Bestrahlungsvorrichtung 1 auf einer horizontalen Fläche oder zum Anhängen der Bestrahlungsvorrichtung 1 an einer vertikalen und/oder auf oder unter einer horizontalen Fläche. In einer weiter bevorzugten Ausführungsform umfaßt der Pendelhalter 15a eine rechtwinklige Halterfläche 15a1 mit einer Länge geringfügig über der Länge des Gehäuses 11 der Bestrahlungsvorrichtung 1 und auf jeder der kurzen Seiten der Halterfläche 15a1 je eine im rechten Winkel zu der Halterfläche 15a1 angeordnete Halterlasche 15a2, 15a3 mit je einem Befestigungsmittel 15a4 zum reversiblen Befestigen der Bestrahlungsvorrichtung(en) 1. Das Befestigungsmittel kann bevorzugt (nicht jedoch beschränkend) eine Rändelschraube (gegebenenfalls mit einer Sicherung) sein, die durch jeweils eine Öffnung in den Haltelaschen 15a2, 15a3 geführt wird und ein Schwenken der Bestrahlungsvorrichtung um die durch die beiden Schrauben gebildeten Drehachse und ein anschließendes reversibles Feststellen in Zusammenwirken mit herkömmlichen Feststellscheiben erlaubt. Dadurch läßt sich die optimale Position der Bestrahlungsvorrichtung relativ zu dem zu bestrahlenden Benutzer leicht und zuverlässig einstellen.

Erfindungsgemäß bevorzugte Beispiele von Ganzkörper-Belichtungseinheiten 100 sind in den Figuren 1C, 3, 4, 6, 7 und 8 gezeigt, auf die nachfolgend Bezug genommen wird.

Die einfachste Ausführungsform einer Ganzkörper-Belichtungseinheit 100 ist in Figur 1C und 3 gezeigt: Die Bestrahlungsvorrichtung ist mit einem (im Wesentlichen kubischen) Standfuß 15b gezeigt, der (in diesem Fall, jedoch nicht beschränkend) einstückig und integral mit der Bestrahlungsvorrichtung 1 ausgeführt ist. Der kubische Standfuß 15b dient einem sicheren Aufstellen der Bestrahlungsvorrichtung 1 und kann zusätzliche Funktionen (wie beispielsweise das Unterbringen von für den Betrieb der Bestrahlungsvorrichtung erforderlichen Einrichtungen, wie oben beschrieben) übernehmen. Einem sicheren Stand, selbst auf einer nicht ebenen Fläche, dienen (aus dem Stand der Technik bekannte) beispielsweise durch Drehen um ein Gewinde in der Höhe variierbare Standfüße, die vorzugsweise aus einem rutschfesten Material (z. B. Kautschuk oder einem Kunststoff) bestehen können.

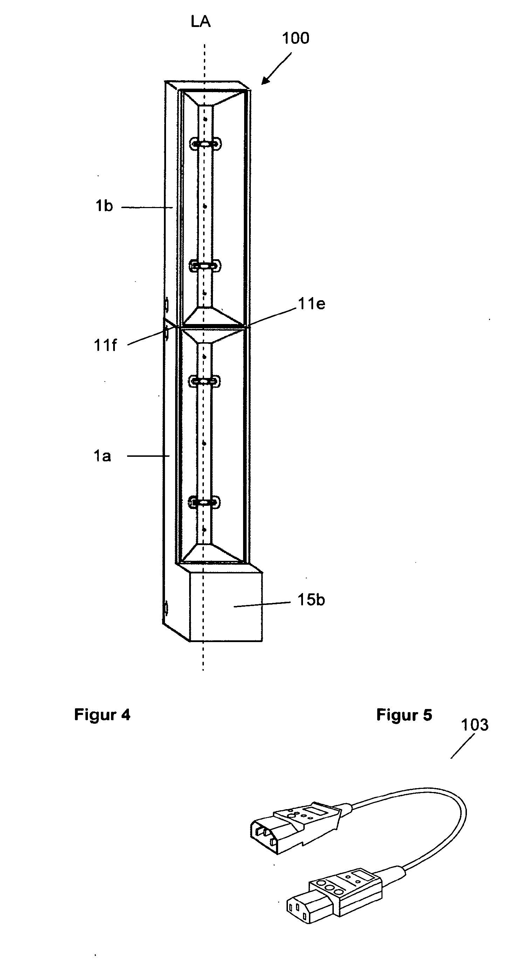

In der in Figur 4 gezeigten bevorzugten Ausführungsform einer Ganzkörper-Belichtungseinheit 100 sind zwei Bestrahlungsvorrichtungen 1 a, 1 b übereinander angeordnet, wobei die obere Wand 11 e des Gehäuses 11 der unteren Bestrahlungsvorrichtung 1a als Basis für den Stand der unteren Wand 11f der oberen Bestrahlungsvorrichtung 1 b dient.

Dabei steht die untere der beiden Bestrahlungsvorrichtungen 1a über den im Wesentlichen kubischen Standfuß 15b auf dem Grund, während die obere der beiden Bestrahlungsvorrichtungen 11 b entweder ungesichert oder (vorzugsweise, aber nicht beschränkend) gesichert auf der oberen Wand 11e der unteren Bestrahlungsvorrichtung steht.

In einer bevorzugten Ausführungsform besteht die Sicherung des Stands des Paares von Bestrahlungsvorrichtungen gemäß Figur 4 aus einem oder zwei (vorzugsweise lösbaren) versenkten Scharnieren 15c1, 15c2, an der jeweiligen Vorderkante der beiden in Kontakt miteinander stehenden Ober- und Unterseiten der beiden Bestrahlungsvorrichtungen 1 a, 1 b und einer gegen das Schwenken/Kippen sichernden Sicherung, beispielsweise einem Verschlußriegel oder einer Verschlußlasche, auf der Rückseite bzw. an der rückwärtigen Kante der beiden in Kontakt miteinander stehenden Seiten. Mittels dieser Sicherung kann ein sicherer Stand der beiden Bestrahlungsvorrichtungen 1 a, 1 b aufeinander gewährleistet werden. In Funktion, also bei Bestrahlen eines Benutzers, bilden diese beiden Bestrahlungsvorrichtungen zwei in die gleiche Richtung strahlende Bestrahlungsvorrichtungen 1 a, 1 b.

Für einen Transport eines solchen Paars von Bestrahlungsvorrichtungen 1 a, 1 b können diese in eine Konfiguration gebracht werden, die sich beispielsweise aus Figur 6 ergibt: Die Kipp-Sicherung auf der Rückseite kann gelöst werden, und die obere Bestrahlungsvorrichtung 1b wird mittels des/der Scharnier(e) 15c1, 15c2 um die Vorderkante der beiden Gehäuse 11 gegen die untere Bestrahlungsvorrichtung 1 a geschwenkt. Im Ergebnis kommen also die beiden Reflektor-Flächen der beiden Bestrahlungsvorrichtungen 1a, 1b gegeneinander zu liegen. Diese Konfiguration kann auf der Vorderseite mittels geeigneter Sicherungs-Einrichtungen (z. B. einer Lasche mit einer Rändelschraube) gesichert werden, so daß die beiden Bestrahlungsvorrichtungen 1 a, 1 b sich nicht voneinander lösen können. Außer Funktion sind also in dieser Konfiguration die beiden Bestrahlungsvorrichtungen 1a, 1b der Ganzkörper-Belichtungseinheit 100 gemäß Figur 6 sicher transportierbar und lagerbar.

Dem Transportieren dienlich könne beispielsweise an geeigneter Stelle montierbare und demontierbare (oder fest installierte) Handgriffe 101 a oder Tragegriffe 101 a sein.

Eine weitere bevorzugt Ausführungsform einer erfindungsgemäßen Ganzkörper-Belichtungseinheit 100 ist aus Figur 7 ersichtlich. Diese umfaßt, vorzugsweise besteht aus, die/den folgende(n) Komponenten:
Mehrere, vorzugsweise drei, Einheiten 105 aus jeweils zwei Bestrahlungsvorrichtungen 1 a, 1 b. Grundsätzlich sind auch Einheiten von drei Bestrahlungsvorrichtungen denkbar, jedoch weniger bevorzugt. Die Paare von Bestrahlungsvorrichtungen sind angeordnet - wie vorstehend beschrieben - jeweils auf einem im Wesentlichen kubischen Standfuß 15b. Vorzugsweise sind die jeweiligen beiden Bestrahlungsvorrichtungen 1 a, 1 b jeder der Einheiten 105a, 105b, 105c auf ihren Endflächen übereinander angeordnet. Sie sind weiter elektrisch und hinsichtlich der aktinischen Strahlung wenigstens einer Wellenlänge gesteuert miteinander verbunden. Sie sind weiter auf der Vorderseite der in Kontakt stehenden Endflächen (obere Endfläche 11e der unteren Bestrahlungsvorrichtung 1 a und untere Endfläche 11f der oberen Bestrahlungsvorrichtung 1 b) mittels eines oder mehrerer versenkbarer Scharniere(s) 15c1, 15c2 lösbar und über die Vorderkante schwenkbar mechanisch verbunden sind. Im Ergebnis sind diese Beiden Bestrahlungsvorrichtungen 1a und 1b in Funktion jeweils zwei in die gleiche Richtung strahlende Bestrahlungsvorrichtungen 1a, 1b einer Einheit 105 sind.

Für einen Ganzkörper-Bestrahlungsvorgang können die mehreren, vorzugsweise drei, Einheiten 105a, 105b, 105c aus jeweils zwei Bestrahlungsvorrichtungen 1a, 1b so, wie in Figur 7 abgebildet, in jeweils zueinander gleichen Abständen angeordnet sein, vorzugsweise an den beiden Endpunkten einer Geraden (bei zwei Paaren von Bestrahlungsvorrichtungen 1 a, 1 b) oder an den Eckpunkten eines Vielecks (bei vielen Bestrahlungsvorrichtungs-Paaren), weiter vorzugsweise an den Eckpunkten eines Dreiecks (bei drei Paaren von Bestrahlungsvorrichtungen; siehe Figur 7). Im Ergebnis bestrahlen sie einen zentral positionierten Benutzer mit der auf diesen gerichteten aktinischen Strahlung wenigstens einer Wellenlänge allseitig, beispielsweise in der in Figur 7 gezeigten Anordnung von drei Seiten.

Figur 8 zeigt eine weitere bevorzugte Ausführungsform einer Ganzkörper-Belichtungseinrichtung 100 gemäß der Erfindung, die auf der in Figur 7 gezeigten Anordnung aufbaut und dieselben Komponenten enthält, wie sie für die Anordnung von Figur 7 beschrieben wurden. Auf deren detaillierte wiederholte Beschreibung kann also an dieser Stelle verzichtet werden.

Wie auf Figur 8 ersichtlich, umfaßt die Ganzkörper-Belichtungseinheit 100 mit drei Paaren/Einheiten 105a, 105b, 105c aus jeweils zwei Bestrahlungsvorrichtungen 1 a, 1 b zusätzlich eine oder mehrere Einrichtung(en) 104a, 104b, 104c, 104d zum Abschirmen der von den Lichtquellen 12a, 12b emittierten aktinischen Strahlung wenigstens einer Wellenlänge. Diese Abschirm-Einrichtungen schaffen zusätzlich zu dem Effekt der Abschirmung der Strahlung gegenüber der Umgebung auch einen (zumindest hinsichtlich der Sicht) abgeschirmten "geschlossenen" Bestrahlungs- bzw. Behandlungsraum und ermöglichen damit auch die für eine Bestrahlung erforderliche Intimität eines abgeschlossenen Raumes.

Erfindungsgemäß ist das Material der Abschirm-Einrichtungen 104a, 104b, 104c, 104d für die emittierte Strahlung undurchlässig oder nur geringfügig durchlässig. Die Abschirm-Einrichtungen 104a, 104b, 104c, 104d sind - vorzugsweise reversibel - an der oder in der Nähe der einen oder mehreren Bestrahlungsvorrichtung(en) 1 befestigbar.

In der in Figur 8 gezeigten beispielhaften Ausführungsform umfassen die Abschirm-Einrichtungen 104a, 104b, 104c, 104d vorzugsweise mobile Stellwände aus starren Ständern aus einem leichten, starren Material (beispielsweise, aber nicht beschränkend: Leichtmetall-/Aluminium-Rohre) mit Stellschrauben-Füßen mit Kautschuk-Unterteil) als aufstellbarem Rahmen mit Einrichtungen zum flexiblen Verbinden der einzelnen Paneele und einem die von den Lichtquellen emittierte aktinische Strahlung wenigstens einer Wellenlänge nicht passieren lassenden, für die Sicht von außen undurchlässigen und idealerweise auch nicht entflammbaren Material aus der Gruppe Textilstoffe oder Polymer-Materialien. Die mobilen Stellwände sind untereinander und in Bezug auf die einbezogenen Bestrahlungsvorrichtungen 1 ohne Werkzeug, einfach, idealerweise von Hand lösbar bzw. befestigbar. Vorzugsweise sind die mobilen Stellwände lösbar mittels Scharnieren, Montageprofilen und Rändelschrauben an den Gehäusen (11) der Bestrahlungsvorrichtungen (1) befestigbar.

In der in Figur 8 gezeigten bevorzugten Ausführungsform ist mittels der Abschirm-Einrichtungen 104a, 104b, 104c, 104d und der Einheiten 105a, 105b, 105c aus jeweils zwei Bestrahlungsvorrichtungen 1 a, 1 b ein geschlossener, über ein schwenkbares Stellwand-Paneel betretbarer Raum gebildet. Dieser ermöglicht ein Abschirmen der Umgebung gegenüber der von den Lichtquellen 12a, 12b der Bestrahlungsvorrichtungen 1 emittierten Strahlung wenigstens einer Wellenlänge. Gleichzeitig ermöglicht diese Ausführungsform ein von der Sicht der Umgebung unbeeinträchtigtes Bestrahlen des Benutzers innerhalb des von den mobilen Stellwänden gebildeten abgeschlossenen Raums.

Die Steuerung einer Bestrahlungsvorrichtung 1 oder mehrerer, zu einer Teilkörper- oder Ganzkörper-Bestrahlungseinheiten 100 bzw. 200 zusammengefaßter Bestrahlungsvorrichtungen kann manuell entweder über den Benutzer selbst oder über eine externe Aufsichtsperson erfolgen. Letzteres wird dann ratsam sein, wenn eine Bestrahlungsbehandlung mit medizinischen Aspekten erwünscht ist. Bevorzugt ist erfindungsgemäß eine Steuerung mittels einer Handbedien-Einheit, die eine elektronische Inbetriebsetzung bzw. Abschaltung (einschließlich Notabschaltung) sowie eine elektronische Steuerung der Bestrahlungszeit und -Intensität erlaubt. Ein (nicht beschränkendes) Beispiel einer solchen Handbedien-Einheit ist beispielhaft in Figur 10 gezeigt. Eine solche Bedien-Einheit ist vorzugsweise mit einer zentralen elektronischen Steuerung aller Parameter aller beteiligten Geräte verbunden und erlaubt auch eine ZeitSteuerung der Bestrahlungsbehandlung bei Einstrahlen von aktinischer Strahlung, die bei Überexposition des Benutzers zu Gesundheitsschäden führt, beispielsweise bei UV-Bestrahlung zu einem Erythem.

Behandlungen mit Betrahlungsvorrichtungen 1 gemäß der Erfindung können in einer Vielzahl von kosmetischen oder medizinischen Zuständen eingesetzt werden. So sind Bestrahlungen beispielsweise zur Behandlung der folgenden medizinischen Zustände indiziert: Behandlung von Psoriasis, insbesondere Psoriasis vulgaris, Psoriasis inversa und anderer einer Lichttherapie zugänglicher Dermatosen; Behandlung der atopischen Dermatitis (Neurodermitis), Akne sowie vergleichbarer, einer Lichttherapie zugänglicher Dermatosen; Behandlung von saisonal bedingten Depressionsformen (SAD), bei Schlafstörungen und immunologischen Funktionsstörungen, die auf zirkadianen und saisonalen Rhythmusstörungen beruhen; zur Verminderung von Vitamin-Mangelzuständen, insbesondere Vitamin-D₃-Mangelzuständen oder zur Anregung von natürlich im Körper vorkommenden oder extern topisch bzw. intern injiziert oder oral verabreichten Photosensibilisatoren.

Die wesentlichen Vorteile der Bestrahlungsvorrichtungen und Belichtungseinheiten der vorliegenden Erfindung liegen in deren geringem Platzbedarf und geringen Gewicht der Geräte, der leichten und sicheren Steuerung und Handhabung, der wahlweisen und bedarfsgerechten Steuerungsmöglichkeiten, der Variabilität der Lichtquellen, der problemlosen Aufstellung der Einheiten und der hohen Mobilität durch kompakten Transport.

Die Erfindung ist nicht auf die in den Figuren gezeigten und in der Beschreibung erläuterten bevorzugten Ausführungsformen beschränkt. Ihr Schutzumfang ergibt sich aus den nachfolgenden Patentansprüchen.

## Patentansprüche

1. Bestrahlungsvorrichtung (1) zum Bestrahlen eines Benutzers mit aktinischer Strahlung, umfassend
- ein quaderförmiges Gehäuse (11) mit fünf Wänden, wobei die fehlende sechste Wand des Quaders die Längswand ist, in deren Richtung - gesehen von der zentralen Längsachse (LA) des Quaders -
- aktinische Strahlung emittiert wird von wenigstens einer austauschbaren, aktinische Strahlung mindestens einer Wellenlänge emittierenden Lichtquelle (12a) und
- die von der wenigstens einen Lichtquelle (12a) emittierte aktinische Strahlung reflektiert wird von einem Reflektor (13) mit mehreren in einer Prisma-Anordnung zueinander angeordneten Reflektorflächen (13a, 13b, 13c, 13d, 13e), von denen mindestens zwei einen Durchlass oder mehrere Durchlässe (14) für Halterungen und Anschlüsse zur Steuerung der wenigstens einen Lichtquelle (12a) und deren Versorgung mit elektrischem Strom aufweisen;
- wobei in dem quaderförmigen Gehäuse (11) Vorschalteinrichtungen, Steuereinrichtungen und Anschlüsse für die Stromversorgung der wenigstens einen Lichtquelle (12a) der Bestrahlungsvorrichtung (1) untergebracht sind; und
- Einrichtungen (15) zum Positionieren der Bestrahlungsvorrichtung (1) relativ zu einem Benutzer.

2. Bestrahlungsvorrichtung (1) nach Anspruch 1, worin das quaderförmige Gehäuse (11) Wände aus einem Material umfaßt, das gewählt ist aus leichten und Stabilität und Standfestigkeit erbringenden Materialien und Kombinationen von Materialien aus der Gruppe Holz, Kunststoff, Leichtmetalle, bevorzugt aus der Gruppe Kunststoff und Leichtmetalle.

3. Bestrahlungsvorrichtung (1) nach Anspruch 1 oder Anspruch 2, umfassend eine, zwei, drei oder vier aktinische Strahlung emittierende Lichtquelle(n) (12a, 12b, 12c, 12d), vorzugsweise eine, zwei, drei oder vier aktinische Strahlung des gleichen Wellenlängenbereichs emittierende Lichtquelle(n) (12a, 12b, 12c, 12d), noch weiter bevorzugt zwei aktinische Strahlung des gleichen Wellenlängenbereichs emittierende Lichtquellen (12a, 12b).

4. Bestrahlungsvorrichtung (1) nach Anspruch 3, umfassend wenigstens zwei aktinische Strahlung in wenigstens zwei Wellenlängenbereichen emittierende Lichtquellen (12a, 12b).

5. Bestrahlungsvorrichtung (1) nach Anspruch 3 oder Anspruch 4, worin der Wellenlängenbereich oder die Wellenlängenbereiche gewählt ist/sind aus der Gruppe Wellenlängenbereich UV-Lichtstrahlung und/oder Wellenlängenbereich sichtbare Lichtstrahlung und/oder Wellenlängenbereich IR-Lichtstrahlung, vorzugsweise aus der Gruppe Wellenlängenbereich von 285 nm bis 400 nm, Wellenlängenbereich von 400 bis 800 nm und/oder Wellenlängenbereich von 800 nm bis 1200 nm; weiter bevorzugt gewählt ist/sind aus der Gruppe des UV-Wellenlängenbereiches von 285 nm bis 350 nm und/oder des UV-Wellenlängenbereiches von 350 nm bis 400 nm und/oder des sichtbaren Wellenlängenbereiches von 400 nm bis 460 nm (violett über blau bis grün), 460 nm bis 550 nm (grün bis gelb), 550 nm bis 680 nm (gelb bis rot) und 680 nm bis 800 nm (rot bis dunkelrot) und/oder der IR-Wellenlängenbereiches von 800 nm bis 1200 nm.

6. Bestrahlungsvorrichtung (1) nach irgendeinem der Ansprüche 1 bis 5, worin der Reflektor fünf Reflektorflächen (13a, 13b, 13c, 13d, 13e) aufweist, von denen sich eine zentrale rechtwinklige Reflektorfläche (13b) und zwei flankierende trapezförmige Reflektorflächen (13a, 13c) in Richtung parallel zu der Quader-Längsachse (LA) erstrecken und in einem Winkel zueinander angeordnet sind, der einem gleichschenkligen Trapez entspricht, und sich zwei weitere trapezförmige Reflektorflächen (13d, 13e) von den oberen bzw. unteren Quader-Endflächen zu den Oberkanten bzw. Unterkanten der zentralen rechteckigen Reflektorfläche (13b) und der beiden trapezförmigen Reflektorflächen (13a, 13c) erstrecken.

7. Bestrahlungsvorrichtung (1) nach irgendeinem der Ansprüche 1 bis 6, worin mehrere, vorzugsweise jeweils zwei, Durchlässe (14a, 14b) für Halterungen und Anschlüsse zur Steuerung der wenigstens zwei Lichtquellen (12a, 12b) und deren Versorgung mit elektrischem Strom an den flankierenden Reflektorflächen (13a, 13c) vorgesehen sind.

8. Bestrahlungsvorrichtung (1) nach irgendeinem der Ansprüche 1 bis 7, umfassend zum Positionieren der Bestrahlungsvorrichtung (1) relativ zu einem Benutzer einen Pendelhalter (15a) zum Aufstellen der Bestrahlungsvorrichtung (1) auf einer horizontalen Fläche oder zum Anhängen der Bestrahlungsvorrichtung (1) an einer vertikalen und/oder horizontalen Fläche, wobei vorzugsweise der Pendelhalter (15a) eine rechtwinklige Halterfläche (15a1) mit einer Länge geringfügig über der Länge der Bestrahlungsvorrichtung und auf jeder der kurzen Seiten je eine im rechten Winkel zu der Halterfläche (15a1) angeordnete Halterlasche (15a2, 15a3) mit je einem Befestigungsmittel (15a4) zum reversiblen Befestigen der Bestrahlungsvorrichtung (1) umfaßt.

9. Bestrahlungsvorrichtung (1) nach irgendeinem der Ansprüche 1 bis 7, umfassend zum Positionieren der Bestrahlungsvorrichtung (1) relativ zu einem Benutzer einen im Wesentlichen kubischen Standfuß (15b), auf dem die Bestrahlungsvorrichtung (1) mit ihrer unteren Endfläche steht oder der mit der unteren Endfläche der Bestrahlungsvorrichtung (1) einstückig ausgebildet ist.

10. Bestrahlungsvorrichtung (1) nach Anspruch 9, worin der Standfuß (15b) zur Unterbringung von Vorschalteinrichtungen, Steuereinrichtungen und/oder Anschlüssen für die Stromversorgung der wenigstens zwei Lichtquellen (12a, 12b) der Bestrahlungsvorrichtung (1) dienen kann.

11. Bestrahlungsvorrichtung (1) nach irgendeinem der Ansprüche 1 bis 10, worin das quaderförmige Gehäuse (11) an einer Gehäusewand oder an mehreren Gehäusewänden Lüftungsschlitze umfaßt.

12. Ganzkörper-Belichtungseinheit (100) oder Teilkörper-Belichtungseinheit (200), umfassend mindestens eine Bestrahlungsvorrichtung (1) nach irgendeinem der Ansprüche 1 bis 11 und eine oder mehrere Einrichtung(en) (101, 102, 103, 104, 106) zum Transportieren, Sichern, Aufstellen, Betreiben und/oder Abschirmen der mindestens einen Bestrahlungsvorrichtung (1).

13. Ganzkörper-Belichtungseinheit (100) nach Anspruch 12, umfassend
- als eine oder mehrere Einrichtung(en) (101) zum Transportieren einen oder mehrere Trage-Einrichtungen (101a), vorzugsweise einen oder mehrere Handgriff(e), besonders bevorzugt einen oder mehrere montierbare(n) und demontierbare(n) Handgriff(e) (101a); und/oder
- als eine oder mehrere Einrichtung(en) (102) zum Sichern einen oder mehrere Fixierlasche(n), Verschlußriegel, Magnetverschluß/-verschlüsse, Rändelmutter(n) (102); und/oder
- als eine oder mehrere Einrichtung(en) (106) zum Aufstellen eine oder mehrere Scharnier(e), Standfüße, Sicherungen (106a); und/oder
- als eine oder mehrere Einrichtung(en) (103) zum Betreiben ein oder mehrere Kabelverbindung(en), Handbedien-Gerät(e) (103a); und/oder
- als eine oder mehrere Einrichtung(en) (104) zum Abschirmen eine oder mehrere für die emittierte Strahlung undurchlässige Abschirm-Einrichtung(en) (104a), vorzugsweise mobile Abschirmeinrichtung(en) (104a, 104b, 104c) zur reversiblen Befestigung an der oder in der Nähe der einen oder mehreren Bestrahlungsvorrichtung(en) (1).

14. Ganzkörper-Belichtungseinheit (100) nach Anspruch 12 oder Anspruch 13, umfassend zwei Bestrahlungsvorrichtungen (1a, 1b) auf einem im Wesentlichen kubischen Standfuß (15b), wobei die beiden Bestrahlungsvorrichtungen (1a, 1b) auf ihren Endflächen übereinander angeordnet sind, elektrisch und hinsichtlich der aktinischen Strahlung wenigstens einer Wellenlänge gesteuert miteinander verbunden sind und auf der Vorderseite der in Kontakt stehenden Endflächen mittels eines oder mehrerer versenkter Scharniere(s) (15c1, 15c2) lösbar und über die Vorderkante schwenkbar mechanisch verbunden sind und in Funktion zwei in die gleiche Richtung strahlende Bestrahlungsvorrichtungen (1a, 1 b) sind und im abgeschalteten oder Ruhe-Zustand zwei über das/die Scharnier(e) (15c1, 15c2) gegeneinander geschwenkte und verbundene Bestrahlungsvorrichtungen (1 a, 1 b) außer Funktion sind.

15. Ganzkörper-Belichtungseinheit (100) nach Anspruch 12 oder Anspruch 13, umfassend
- mehrere, vorzugsweise drei, Einheiten (105) aus jeweils zwei Bestrahlungsvorrichtungen (1a, 1b), jeweils auf einem im Wesentlichen kubischen Standfuß (15b), wobei die jeweiligen beiden Bestrahlungsvorrichtungen (1a, 1b) jeder der Einheiten (105a, 105b, 105c) auf ihren Endflächen übereinander angeordnet sind, elektrisch und hinsichtlich der aktinischen Strahlung wenigstens einer Wellenlänge gesteuert miteinander verbunden sind und auf der Vorderseite der in Kontakt stehenden Endflächen mittels eines oder mehrerer versenkter Scharniere(s) (15c1, 15c2) lösbar und über die Vorderkante schwenkbar mechanisch verbunden sind und in Funktion jeweils zwei in die gleiche Richtung strahlende Bestrahlungsvorrichtungen (1 a, 1 b) einer Einheit (105) sind, und die mehreren, vorzugsweise drei, Einheiten (105a, 105b, 105c) aus jeweils zwei Bestrahlungsvorrichtungen (1 a, 1 b) in jeweils zueinander gleichen Abständen, vorzugsweise an den beiden Endpunkten einer Gerade oder an den Eckpunkten eines Vielecks, weiter vorzugsweise an den Eckpunkten eines Dreiecks, so angeordnet sind, daß sie einen zentral positionierten Benutzer mit der auf diesen gerichteten aktinischen Strahlung wenigstens einer Wellenlänge allseitig bestrahlen; und
- eine oder mehrere Einrichtung(en) (104a, 104b, 104c) zum Abschirmen der von den Lichtquellen (12a, 12b) emittierten aktinischen Strahlung wenigstens einer Wellenlänge, die für die emittierte Strahlung undurchlässig ist/sind und vorzugsweise reversibel an der oder in der Nähe der einen oder mehreren Bestrahlungsvorrichtung(en) (1) befestigbar ist/sind.

16. Ganzkörper-Belichtungseinheit (100) nach Anspruch 15, worin die Abschirm-Einrichtung(en) (104a, 104b, 104c) mobile Stellwände aus starren Ständern und einem die emittierte aktinische Strahlung wenigstens einer Wellenlänge nicht passieren lassenden und für die Sicht von außen undurchlässigen Material aus der Gruppe Textilstoffe und PolymerMaterialien sind und lösbar an den Bestrahlungsvorrichtungen befestigbar sind, vorzugsweise lösbar mittels Scharnieren, Montageprofilen und Rändelschrauben an den Gehäusen (11) der Bestrahlungsvorrichtungen (1) befestigbar sind.

17. Teilkörper-Belichtungseinheit (200) nach Anspruch 12, umfassend
- mindestens eine, vorzugsweise eine, Bestrahlungsvorrichtung (1) nach irgendeinem der Ansprüche 1 bis 11; und
- zum Positionieren der Bestrahlungsvorrichtung (1) relativ zu einem Benutzer einen Pendelhalter (15a) zum Aufstellen der Bestrahlungsvorrichtung (1) auf einer horizontalen Fläche oder zum Anhängen der Bestrahlungsvorrichtung (1) an einer vertikalen und/oder horizontalen Fläche, wobei vorzugsweise der Pendelhalter (15a) eine rechtwinklige Halterfläche (15a1) mit einer Länge geringfügig über der Länge der Bestrahlungsvorrichtung und auf jeder der kurzen Seiten je eine im rechten Winkel zu der Halterfläche (15a1) angeordnete Halterlasche (15a2, 15a3) mit je einem Befestigungsmittel (15a4) zum reversiblen Befestigen der Bestrahlungsvorrichtung(en) (1) umfaßt.
